# EUROPEAN PATENT APPLICATION

(11) **EP 3 669 978 A1**
(43) Date of publication of application: **24.06.2020**
(21) Application number: 18306736.2
(22) Date of filing: 19.12.2018
(51) Int. Cl.: B01J 31/16, B01J 31/18, B01J 35/00, C07D 257/08

(54) **BI-FUNCTIONAL NANOHYBRIDS**

(71) Applicant: Université de Bourgogne, 21078 Dijon Cedex (FR)
(72) Inventor: DECREAU, Richard, 21000 DIJON (FR); DOULAIN, Pierre-Emmanuel, 21000 DIJON (FR); PARIS, Jérémy, 21000 DIJON (FR)
(74) Representative: IPAZ

(57) **Abstract**

Bi-functional nanohybrids comprising a nanoparticle to the surface of which are covalently coupled chemical functions, one of which being biorthogonal, and their use as support for catalysts

## Description

The present invention relates to bi-functional nanohybrids, to catalysts built up from these nanohybrids, to processes for the production of these nanohybrids and to the use of said nanohybrids in catalytic processes.

Catalysis in industry is widely used for the synthesis of chemicals in large quantities (about 90% of chemical processes are catalysed).

Three types of catalysis exist:
- Homogeneous catalysis refers to catalysts that are in the same phase as the reagents. This is very powerful and may catalyse a wide range of reactions. Its major disadvantage is the difficulty of obtaining the pure final product, i.e., separated from all the elements used in the synthesis.
- Heterogeneous catalysis refers to catalysts in a different phase than reagents. For example, palladium on carbon (Pd/C) is widely used in the chemical industry to catalyse hydrogenation reactions. Its advantage lies in its very practical implementation. Its disadvantage relates to its use which is limited to certain chemical reactions. Thus, asymmetric syntheses cannot be catalysed via heterogeneous catalysis.
- Supported catalysis consists in immobilizing homogeneous catalysts on an insoluble solid support. The latter benefits from the selectivity and efficiency of homogeneous catalysis, as well as the simplicity of processing and recyclability of heterogeneous catalysis. However, the supported catalysis may also encounter problems of catalyst deactivation, lower catalytic activity compared to the homogeneous version and release of the metal that exerts the catalytic activity.

In general, it seems that supported catalysts are mainly used in the fine chemical industry where the added value of the product to be synthesized is high. In addition, the supported catalysis is in line with a concern for compliance with environmental standards (simplicity of filtration, less solvent and recycling of metal facilitated which is in line with the principles of green chemistry).

Catalysts may be mono- or multi-functional, in particular bi-functional. A monofunctional catalyst contains only one type of catalytic site, i.e. every catalytic site or surface exhibits the same qualitative catalytic property as to what reaction or reaction its can catalyse. On the contrary in a multi-functional catalyst, each site catalyses different reactions and reaction steps. Many bifunctional catalysts possess either Lewis or Bronsted basic functionality and a hydrogen-bond donor group suitably positioned over a chiral scaffold. Compared to monofunctional group catalysts, the cooperative effect of the two complementary functional groups can lead to new reactivity and new stereocontrol in reactions.

A substantial effort and cost in organic synthesis are often necessary to design catalysts of the monofunctional type usable in homogeneous phase. This is particularly true in the case of monometallic catalyst as soon as one wishes that the sphere of coordination of the metal is complete, which is a guarantee of performance and efficiency for a catalyst. This same synthetic effort is generally necessary to develop bifunctional catalysts, a fortiori if one wants the coordination sphere of each metal to be complete.

Thus mono- and bi-functional systems have been developed in the literature as
- Laccase model catalysts (McCrory, C. C. L.; Devadoss, A.; Ottenwaelder, X.; Lowe, R. D.; Stack, T. D. P.; Chidsey, C. E. D., Electrocatalytic O2 Reduction by Covalently Immobilized Mononuclear Copper(I) Complexes: Evidence for a Binuclear Cu2O2 Intermediate., J. Am. Chem. Soc., 2011, 133, 3696-3699),
- Cytochrome c oxidase models (Collman, J. P.; Devaraj, N. K.; Decréau, R. A.; Yang, Y.; Yan, Y.; Ebina, W.; Eberspacher, T. P. A.; Chidsey. C. E. D, A Functional mimic of cytochrome c oxidase selectively reduces oxygen by four electrons under rate-limiting electron flux: the role of CuB and Tyr-244, Science, 2007, 315, 1565-1568 ; Collman, J. P.; Decréau, R. A.; Zhang, C., Synthesis of Cytochrome c Oxidase Models Bearing a Tyr244 Mimic, J. Org. Chem., 2004, 69, 3546-3549),
- Co salen cofacial binuclear salt catalysts for asymmetric epoxide opening (R. M. Haak, M. Martinez Belmonte, E. C. Escudero-Adan, J. Benet-Buchholz and A. W. Kleij, Olefin metathesis as a tool for multinuclear Co(III)salen catalyst construction: access to cooperative catalysts, Dalton Trans., 2010, 39, 593-602 ; J. M. Ready and E. N. Jacobsen, Cooperative multimetallic catalysis using metallosalens, J. Am. Chem. Soc., 2001, 123, 2687-2688) or for enantioselective nitro-aldol (Henry) reaction (J. Park, K. Lang, K. A. Abboud and S. Hong, Self-Assembled Dinuclear Cobalt(II)-Salen Catalyst Through Hydrogen-Bonding and Its Application to Enantioselective Nitro-Aldol (Henry) Reaction, J. Am. Chem. Soc., 2008, 130, 16484-16485),
- Ruthenium tris-pyridine cofacial binuclear catalysts for water oxidation (Tohru Wada, Shunsuke Nishimura, Taro Mochizuki, Tomohiro Ando and Yuji Miyazato, Mechanism of Water Oxidation Catalyzed by a Dinuclear Ruthenium Complex Bridged by Anthraquinone, Catalysts 2017, 7, 56),
- Pt/Pd binuclear catalysts for Heck coupling (Jones, N. D.; James, B. R, Homo- and Heterobimetallic Precursor Catalysts for the Heck Reaction, and a Proposal for a General Catalytic Cooperativity Index, Adv. Synth. Catal. 2002, 344, 1126),
- Bimetallic catalysts for alkyne activation, and many other bimetallic catalysts for coupling reaction catalysis (Paulin Buchwalter, Jacky Rose and Pierre Braunstein, Multimetallic Catalysis Based on Heterometallic Complexes and Clusters, Chem. Rev. 2015, 115, 28-126).

Furthermore, if such catalysts are to be immobilized, then an additional immobilization step is required, which adds to the synthesis effort. Finally, the number of steps to consider is often too large and prohibitive for researchers who do not wish to engage in a substantial or even large-scale organic synthesis program.

Previous studies have described metalloporphyrins immobilized by coordination binding on electrode or nanoparticle bearing a ligand (Eberspacher, T. A.; Collman, J.P.; Chidsey, C.E.D.; Donohue, D.L.; Van Ryswyk, H., Modular Assembly and Air-Stable Electrochemistry of Ruthenium Porphyrin Monolayers, Langmuir, 2003, 19(9), 3814-3821 ; Saeedi, M. S., Tangestaninejad S., Moghadam M., MirkhaniIraj V., Mohammadpoor-Baltork I., Khosropour R. A., Manganese porphyrin immobilized on magnetite nanoparticles as a recoverable nanocatalyst for epoxidation of olefins, Materials Chemistry and Physics, 2014, 146, 113-120). This coordination bond is potentially labile, and remains dependent in particular on the degree of oxidation of the catalyst and the pH of the medium; thus the catalyst can come off from the particle/surface and then be definitively washed once it is released.

Finally, there are already studies describing metalloporphyrins bearing both a coordination ligand and a chemical function allowing the immobilization on surfaces. However, such synthetic strategies are much longer, tedious and more expensive than that of the present invention that is much more straightforward.

Other previous studies describe bi-nuclear models synthesized by simple immobilization of mononuclear complexes on gold electrodes covered with a self-assembled monolayer coating (McCrory, C. C. L.; Devadoss, A.; Ottenwaelder, X.; Lowe, R. D.; Stack, T. D. P.; Chidsey, C. E. D., Electrocatalytic O2 Reduction by Covalently Immobilized Mononuclear Copper(I) Complexes: Evidence for a Binuclear Cu2O2 Intermediate, J. Am. Chem. Soc., 2011, 133, 3696-3699). The models facing each other thus recreate bi-nuclearity, which is achieved only through the controlled number of binding functions at the surface of an electrode. By addressing the nature of the bioorthogonal function and chemical function of the coating, the invention may allow the modular recreation of other sophisticated bi-nuclear systems, whether the metals are identical, model Cu/Cu, or Fe/Fe, or Mn/Mn or Ru/Ru (R.M. Haak cited above) or different as Fe/Cu (Collman, J. P and R.M. Haak cited above) or whether the ligands of these metals are identical (two tris-pyridyl ligands for example) or different (a porphyrin ligand and a tris-pyridine ligand for example). This same approach can be considered for the design of tri-nuclear species, in particular tri-metallic catalysts for which the synthesis of ligands to obtain a catalyst in homogeneous phase would become a real challenge.

Finally, if the prior art concerning surfaces (particles, electrodes) functionalized by a bioorthogonal function is vast, there are fewer studies for tetrazines and no studies have been made with triazines and mono- and bi-nuclear catalysts.

Thus there is a need for new catalytic processes that would be less expensive, more green and simpler to achieve. Hence, there is a need for catalysts that would be cheap, easy to synthesize and easy to use as immobilized catalysts.

Consequently, the present invention will partly remedy such efforts and such costs by proposing the immobilization on specific bi-functional nanohybrids of inexpensive catalysts that eventually may not be totally functional catalysts because of an incomplete coordination sphere. The structure of the nanohybrids according to the invention offers such an environment, which:
a) will complete the coordination sphere of a mononuclear catalyst, thus making it functional and efficient, or
b) will allow the relative ease of synthesis of bi-nuclear catalysts that will keep each subunit in a cofacial mode without complex organic chemistry designs. This is only possible thanks to the specific design of the nanohybrid according to the invention: the design of this bi-functional nanohybrid is only possible by the very specific design of an organic coating on the nanoparticle surface.

The rationale objective of the invention is to bring a key partner to a catalyst in order to turn on a specific type of activity. Hence, such a partner combined to a given catalyst will allow:
1) To complete the coordination sphere of the catalyst (in such a specific case, the partner is a ligand)
2) To achieve asymmetric catalysis or to resolve a racemic mixture (in such specific cases, the partner is a chiral molecular entity)
3) to make the synthesis of a multi-nuclear catalyst possible (in such a specific case, the partner is another catalyst)
4) To make a given catalyst usable in photo-redox catalysis (in such a specific case, the partner is a photosensitizer)
5) To address the nanohybrid properties and to increase the compatibility of the nanohybrid within its environment. Hence, the partner will make the use of such an immobilized catalyst possible in a given environment for example by adding chemical groups of choice capable of addressing the stability of the nanohybrid. To allow the nanohybrid to be stable in water and its immobilized catalyst to achieve optimized catalytic reactions in water, the partner entity may be for example a molecule bearing water-soluble polar head groups.

Consequently, the first object of the instant invention is a bi-functional nanohybrid comprising a nanoparticle to the surface of which are covalently coupled one or more, identical or different, groups selected from:
- either a group of formula (1) or (1') 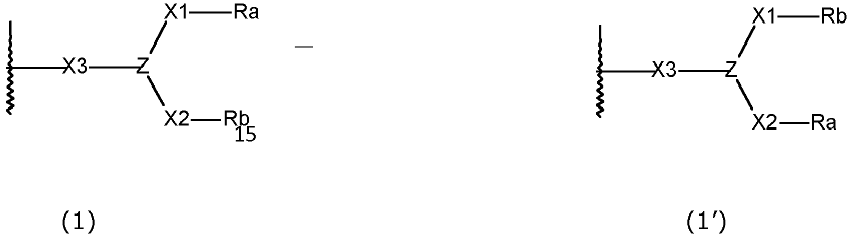 where
   - Ra represents a bioorthogonal function selected from:
      -▪1,2,4-thazines of formula (a) 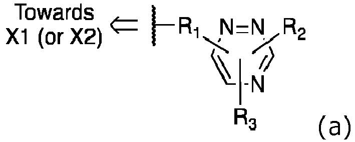 R₁, R₂ and R₃ being independently either in position 3, 5 or 6 of said 1,2,4-triazine
         R₁ being either absent or an aryl or a heteroaryl group. In the case of an aryl or heteroaryl group, these groups have at least one attachment function to X1 or X2, among the positions of the cycle. The following groups: -aryl-COO-, -aryl-O- and -aryl-NH- are examples of R₁ bearing an attachment function. The one skilled in the art will be able to choose said function in view of his general knowledge.
         R₂ and R₃ being selected in the group comprising:
         ✔ hydrogen atom and halogen atoms,
         ✔ -CF₃, -CN, -NO₂ and
         ✔ aryl and heteroaryl group,
      said triazine of formula (a) being bound to X1 or X2 in one of the position 3, 5 or 6 either directly (when R₁ is absent) or via R₁,
      and from
      ▪1,2,4,5-tetrazines of formula (b) 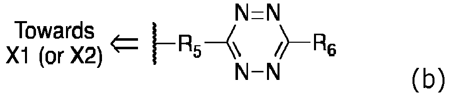
         R₅ being absent or being selected in the group comprising:
            ✔ oxygen and sulphur atoms,
            ✔ -O-aryl and -S-aryl said aryl group bearing an attachment function to X1 or X2 in any free position,
            ✔ -NRm- with Rm being a hydrogen atom, a (C₁-C₄)alkyl group, a (C₃-C₆)cycloalkyl group, an aryl group or a heteroaryl group,
            ✔ -NRm-aryl said aryl group bearing an attachment function to X1 or X2 in any free position and
            ✔ aryl and heteroaryl group, said group bearing an attachment function to X1 or X2 in any free position. As example of group bearing an attachment function to X1 or X2 can be cited the following groups: -aryl-COO-, - aryl-O- and -aryl-NH-. The one skilled in the art will be able to choose said function in view of his general knowledge.
         R₆ being selected in the group comprising:
            ✔ hydrogen atom,
            ✔ (C₁-C₄)alkyl group and (C₃-C₆)cycloalkyl group,
            ✔ -ORm and -SRm with Rm being a hydrogen atom, a (C₁-C₄)alkyl group, a (C₃-C₆)cycloalkyl group, an aryl group or a heteroaryl group,
            ✔ -NRmRo with Rm and Ro being independently from each other a hydrogen atom, a (C₁-C₄)alkyl group, a (C₃-C₆)cycloalkyl group, an aryl group or a heteroaryl group and
            ✔ aryl and heteroaryl group,
      said tetrazine of formula (b) being bound to X1 or X2 either directly (when R₅ is absent) or via R₅
   - Rb represents
      ∘ an halogen atom,
      ∘ a -ORm or -SRm group with Rm being a hydrogen atom, a (C₁-C₄)alkyl group, a (C₃-C₆)cycloalkyl group, an aryl group or a heteroaryl group,
      ∘ a -COORm group with Rn being a hydrogen atom, a (C₁-C₄)alkyl group, a (C₃-C₆)cycloalkyl group, an aryl group or a heteroaryl group,
      ∘ a -NRmRo with Rm and Ro being independently from each other a hydrogen atom, a (C₁-C₄)alkyl group, a (C₃-C₆)cycloalkyl group, an aryl group or a heteroaryl group
      ∘ an azide group,
      ∘ a maleimidyl group,
      ∘ a (C₁-C₄)alkynyl group,
      ∘ an imidazolyl group,
      ∘ a pyridinyl group,
      ∘ a 1,2,4-triazine of formula (a),
      ∘ a 1,2,4,5-tetrazine of formula (b),
      ∘ a diphenylphosphinobenzoate derivative of formula (c) 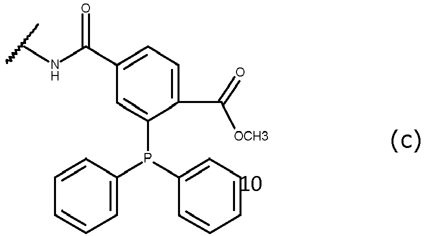 and
      ∘ a norbornene derivative of formula (d) 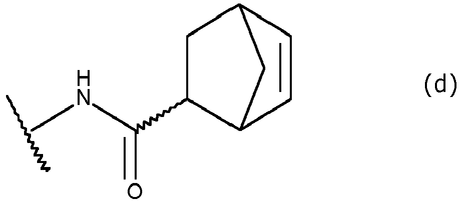
      and
   - X1, X2 and X3, same or different, may be absent or represent a spacer selected from (C₁-C₂₆)alkyl groups; said groups may comprise one or more oxygen atoms and/or one or more groups 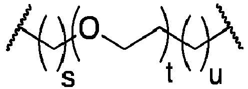 with s, t and u, same or different being integers between 0 and 10,
   - Z represents an aryl group or a heteroaryl group that is at least tri-substituted by substituents allowing the attachment to X1, X2, and X3 respectively. The one skilled in the art will be able to choose said substituents in view of his general knowledge.
- either a group of formula (2) 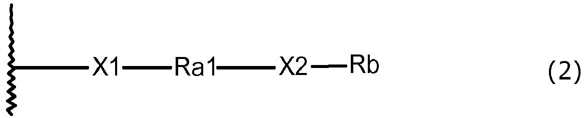 where
   - Ra1 represents a bioorthogonal function selected from:
      ▪1,2,4-triazines of formula (e) 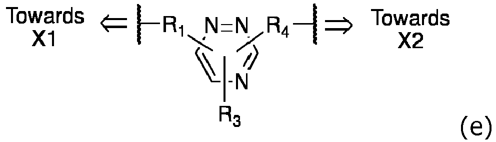 R₁, R₃ and R₄ being independently either in position 3, 5 or 6 of said 1,2,4-triazine
         R₁ and R₃ being as defined above
         R₄ being either absent or an aryl or a heteroaryl group. In the case of an aryl or heteroaryl group, these groups have at least one attachment function to X2, among the positions of the cycle. The following groups: -aryl-COO-, -aryl-O- and -aryl-NH- are examples of R₄ bearing an attachment function. The one skilled in the art will be able to choose said function in view of his general knowledge,
      said triazine being bound respectively to X1 and X2 in two of the positions 3, 5 or 6 either directly (when R₁ or R₄ or both are absent) or via R₁ and R₄, respectively,
      and
      ▪1,2,4,5-tetrazines of formula (f) 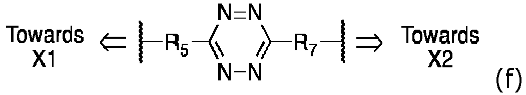
         R₅ being as defined above
         R₇ being selected in the group comprising:
            ✔ oxygen and sulphur atoms,
            ✔ -O-aryl and -S-aryl said aryl group bearing an attachment function to X2 in any free position,
            ✔ -NRm- with Rm being a hydrogen atom, a (C₁-C₄)alkyl group, a (C₃-C₆)cycloalkyl group, an aryl group or a heteroaryl group,
            ✔ -NRm-aryl said aryl group bearing an attachment function to X2 in any free position and
            ✔ aryl and heteroaryl group said group bearing an attachment function to X2 in any free position. As example of group bearing an attachment function to X2 can be cited the following groups: -aryl-COO-, -aryl-O- and -aryl-NH-. The one skilled in the art will be able to choose said function in view of his general knowledge.
      said tetrazine being bound to X1 and X2 either directly (when R₅ or R₇ or both are absent) or via R₅ and R₇, respectively
      and
   - Rb is as defined above
   - X1 and X2 are as defined above
with the proviso that Ra and Rb are mutually compatible, i.e. do not react between each other.

According to the invention, a control of a 1/1 ratio between Ra and Rb or between Ra1 and Rb is made possible.

In the present invention, a (C₁-C₄)alkyl group includes, for example, straight-chain or branched lower alkyl groups having 1 to 4 carbon atoms, such as methyl, ethyl, propyl, isopropyl, butyl, isobutyl and tert-butyl. They may include one or more double and triple bounds and thus include ethylenyl, propylenyl, iso-propylenyl, butylenyl, iso-butylenyl, ethynyl and the like, wherein the divalent and triple bonds may be at any of the carbon atoms of the alkyl group or as specifically indicated.

A (C₁-C₂₆)alkyl group includes, for example, straight-chain or branched alkyl groups having 1 to 26 carbon atoms, or 1 to 7 carbon atoms or 2 to 6 carbon atoms, said chains including optionally one or more double or triple bonds such as methyl, ethyl, propyl, isopropyl, butyl, isobutyl and tert-butyl, pentyl, neopentyl, isopentyl, hexyl, heptyl, octanyl, nonyl, capryl, lauryl, stearyl, eicosyl, hexacosyl, ethylenyl, propylenyl, iso-propylenyl, butylenyl, iso-butylenyl, ethynyl and the like, wherein the divalent and triple bonds may be at any of the carbon atoms of the alkyl group or as specifically indicated. Said group may be substituted.

A (C₃-C₆)cycloalkyl group includes, for example, cycloalkyl groups having 3 to 6 carbon atoms and optionally one or more double and triple bonds, such as cyclopropyl, cyclobutyl and the like. Said group may be substituted.

An alkylenyl group includes, for example, straight or branched alkyl groups having 1 to 20 or 1 to 10 or 1 to 4 carbon atoms and comprising at least one double bond. Exemplary alkylenyl groups include ethylenyl, propylenyl, iso-propylenyl, butylenyl, iso-butylenyl, pentylenyl, 1-methylbutylenyl, 1-ethylpropylenyl, 3-methylpentylenyl, and the like, wherein the divalent bonds may be at any of the carbon atoms of the alkylenyl group, or as specifically indicated. As used herein, "alkylenyl" also includes cycloalkylenyl when three or more carbon atoms are referenced.

A (C₁-C₄)alkynyl group includes monoradicals of an unsaturated hydrocarbon, having at least 1 triple bond. Preferred alkynyl groups include ethynyl, (-C≡CH), propargyl (or propynyl, -C≡CCH3), and the like. Said group may be substituted.

In the present invention, a bioorthogonal function is a chemical function that is not encountered in biomolecules in natural systems, (amine, carboxylic acid, ester, thiols, alcohols, phosphates, etc..) and that does not react with these functions. In the present invention, bioorthogonal functions are designed by Ra and Ra1.

Halogen atoms include chlorine, bromine, iodine and fluorine atoms.

An aryl group includes aromatic hydrocarbons having 6 to 14 carbon atoms. Exemplary aryl groups include phenyl, naphthyl and anthracenyl. The radicals may also be fused to other saturated, (partially) unsaturated or aromatic ring systems. It is possible for the linkage to particles to take place via any possible ring member of the aryl radical. Said group may be substituted.

An heteroaryl group includes 5-, 6- or 7-membered cyclic aromatic radical, which comprises at least 1, where appropriate also 2, 3, 4 or 5, heteroatoms, the heteroatoms being identical or different. It is possible for the linkage to the particles and to A to take place via any possible ring member of the heteroaryl radical. The heterocycle may also be part of a bi- or polycyclic system. Preferred heteroatoms are nitrogen, oxygen and sulphur. It is preferred for the heteroaryl radical to be selected from the group comprising pyrrolyl, furyl, thienyl, thiazolyl, oxazolyl, isoxazolyl, pyrazolyl, imidazolyl, pyridinyl, pyrimidinyl, pyridazinyl, pyrazinyl, phthalazinyl, indolyl, indazolyl, indolizinyl, quinolinyl, isoquinolinyl, quinoxalinyl, quinazolinyl, carbazolyl, phenazinyl, phenothiazinyl, acridinyl. Said group may be substituted.

In the invention, nanoparticle means a natural material, accidentally formed or manufactured containing free particles, in the form of an aggregate or an agglomerate, of which at least 50% of the particles, in the size distribution, have one or more external dimensions between 1 nm and 100 nm. In the present invention, the focus is made on metal oxide-, metal-, and carbon-based nanoparticles.

According to the invention an integer between 0 and 10 means 0, 1, 2, 3, 4, 5, 6, 7, 8, 9, and 10.

According to the invention "covalently coupled" or "covalently bound" are equivalent.

According to the invention, "Ra and Rb are mutually compatible" means that they do not react together and the one skilled in the art will be able to choose them in view of his general knowledge.

In a specific embodiment of the invention, Ra in formula (1) and (1') is a 1,2,4-triazine of formula (a1) 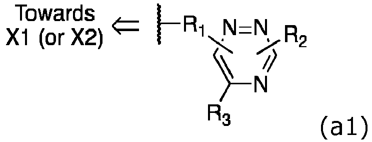 wherein
R₃ is a hydrogen atom and is in position 5 of the said 1,2,4-triazine
R₁ is selected from 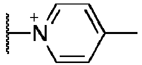 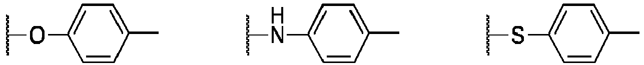 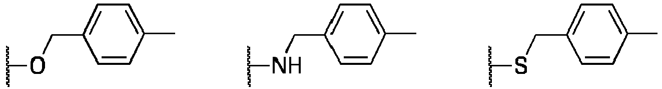 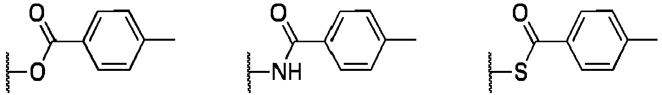
R₂ is selected from:
   ✔ a hydrogen atom
   ✔ a bromine atom, a fluorine atom
   ✔ a -CF₃ group, a -CN group
   ✔ a phenyl group optionally mono- or poly- substituted by a fluorine atom, -OCH₃ group, -CF₃ group, -NO₂ group
R₁ and R₂ being independently either in position 3 or 6 of said 1,2,4-triazine

In another specific embodiment of the invention Ra in formula (1) and (1') is a 1,2,4,5-tetrazine of formula (b1) 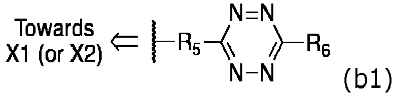 wherein
R₅ is selected from:
   ✔ an oxygen atom, a sulphur atom
   ✔ a -NH- group
   ✔ one of the following groups 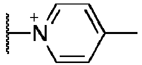 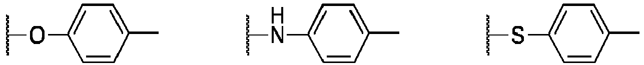 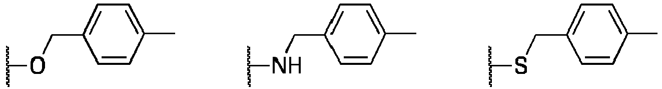 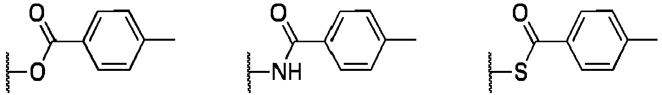
R₆ is selected from:
   ✔ a hydrogen atom,
   ✔ a methyl group,
   ✔ -OCH₃ and -SCH₃,
   ✔ -N(CH₃)₂ and
   ✔ a phenyl group optionally mono- or poly- substituted with a fluorine atom, -OCH₃ group, -CF₃ group, -NO₂ group.

In another embodiment according to the invention, Ra1 of formula (2) corresponds to a 1,2,4-triazine of formula (e1) 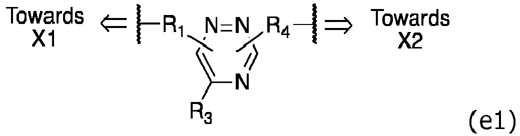 wherein
R₁ is selected from 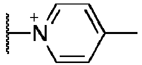 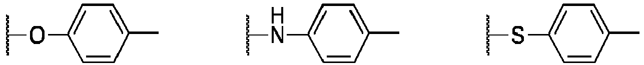 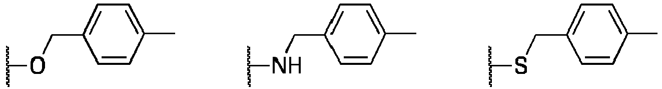 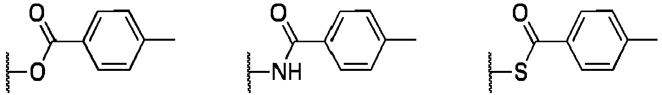
and R₃ is a hydrogen atom and is in position 5 of the said 1,2,4-triazine
R₄ is selected from 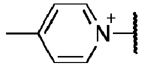 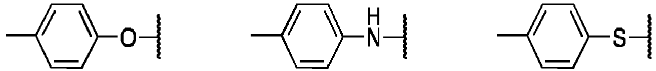 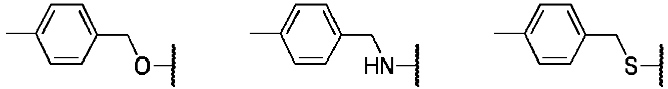 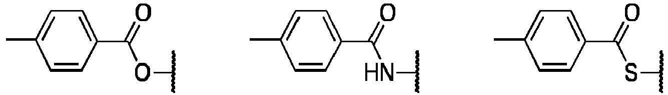
R₁ and R₄ being independently either in position 3 or 6 of said 1,2,4-triazine R₁ and R₄ being the same or different

In another embodiment according to the invention, Ra1 of formula (2) corresponds to a 1,2,4,5-tetrazine of formula (f1) 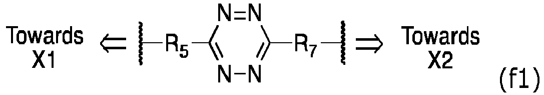 wherein
R₅ is selected from:
   ✔ an oxygen atom, a sulphur atom
   ✔ a -NH- group
   ✔ one of the following groups 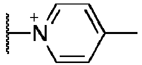 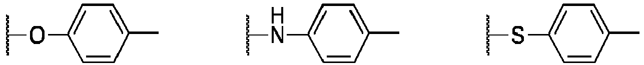 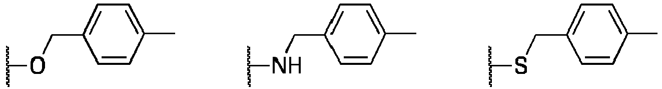 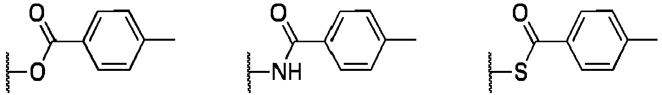
R₇ may be absent or is selected from:
   ✔ an oxygen atom, a sulphur atom
   ✔ a -NH- group
   ✔ one of the following groups 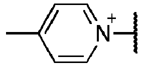 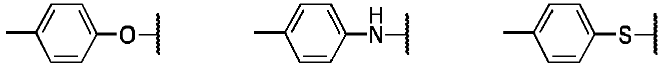 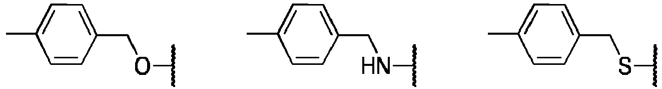 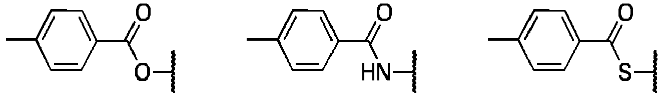
R₅ and R₇ being the same or different

Another object according to the invention is a method for synthesizing a bi-functional nanohybrid according to the invention, comprising the step of attachment on a nanoparticle bearing at its surface a substituent Xa at least one compound of formula (I) 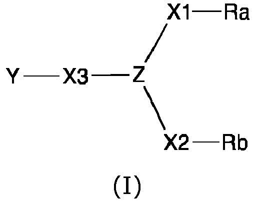 or of formula (I') 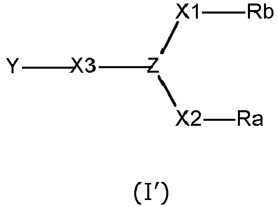 or of formula (II) 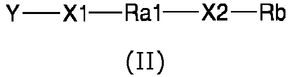 wherein
Ra, Ra1, Rb, X1, X2, X3 and Z are as defined above and
Y represents a functional group, which allows the covalent attachment to the surface of said nanoparticle via Xa and is
   - either a chlorine, a bromine or an iodine atom
   - either a -NRpRq group with Rp and Rq being each independently from the other a hydrogen atom, a (C₁-C₄)alkyl group or a (C₃-C₆)cycloalkyl group,
   - either a carboxylic acid group,
   - either an activated ester group,
   - either a maleimidyl group,
   - either a thiol group,
   - either a hydroxyl group,
   - either a tosylate group,
   - either a triflate group,
   - either a mesylate group,
   - either an acid halide group,
   - either an acid anhydride group,
   - either an isocyanate group,
   - either an isothiocyanate group,
   - either an azide group,
   - either an alkynyl group,
   - either a N-heteroaryl group

An activated ester group includes any ester that can be characterized as having an increased chemical reactivity due to the presence of a better leaving group. Depending upon available reagents, resources and desired results, one skilled in the art may choose the appropriate activated ester.

A triflate group is equivalent to a trifluoromethane sulfonyl group.

A mesylate group is an ester of methanesulfonic acid.

As acid halide group may be cited acyl chloride functional group, such as acetyl chloride functional group.

As acid anhydride group may be cited carboxylic anhydride functional group.

The choice of Xa will be conditioned by the nature of Y and the choice of Y will be conditioned by the nature of Xa, each of them being chosen in order to have the possibility to create between them a covalent bond. The one skilled in the art will be able to make the choice on the basis of his general knowledge.

The covalent bond may be any one of a by classical chemical reactions non-degradable bond. Here, examples of the non-degradable bond may include amine, ammonium, ether, thioether, ester and amide and the like, but the present invention is not necessarily limited thereto. All these bonds are well known from the one skilled in the art.

The step of attachment is realised by known methods in the art as for example the one according to Chem. Comm. 2013, 7394-7396.

Another object of the invention is a compound of formulas (Ia), (Ib), (Ic), (Id) or (Ie) 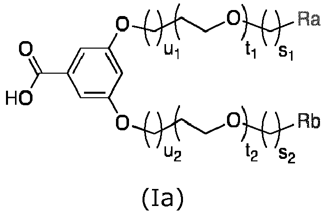 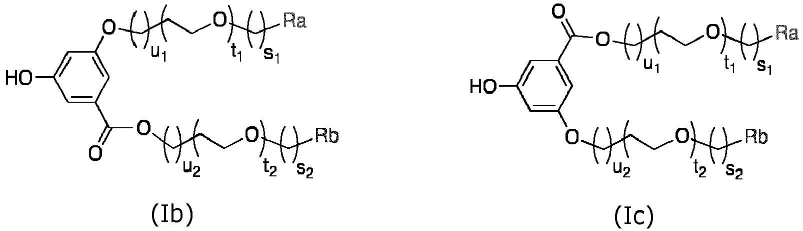 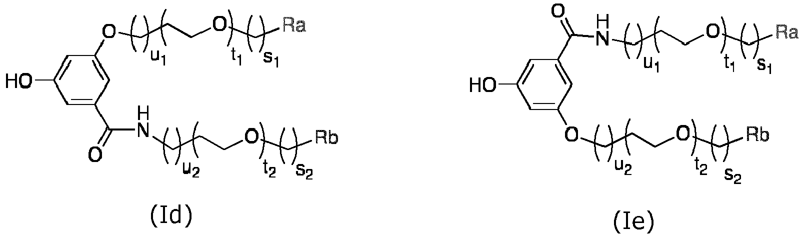 wherein
Ra represents a 1,2,4-triazine of formula (a1) or a 1,2,4,5-tetrazine of formula (b1),
Rb is as defined above,
s₁, t₁, u₁, s₂, t₂ and u₂ same or different are integers between 0 and 10,
Y is a -COOH (Ia) or a -OH (Ib), (Ic), (Id) and (Ie) and
X3 is absent

Another object of the invention is a compound of formula (II) wherein Ra1 is a 1,2,4-triazine corresponds to formula (e1) and corresponding to formula (IIa) 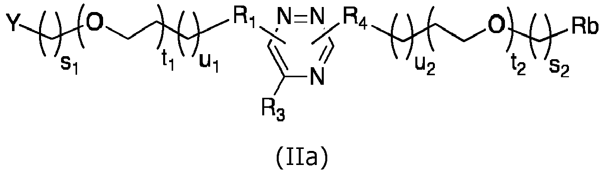 wherein
Y represents
   - either a chlorine, bromine, iodine atoms
   - either a -COOH group,
   - either an activated ester group,
   - either a maleimidyl group,
   - either a -OH, -SH or -NH₂ group
R₁, R₃, R₄ and Rb are as defined above and
s₁, t₁, u₁, s₂, t₂ and u₂ same or different are integers between 0 and 10

Another object of the invention, a compound of formula (II) wherein Ra1 is a 1,2,4,5-tetrazine corresponds to formula (f1) and corresponding to formula (IIb) 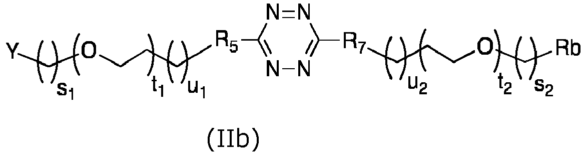 wherein
Y represents
   - either a chlorine, bromine, iodine atoms
   - either a -COOH group,
   - either an activated ester group,
   - either a maleimidyl group,
   - either a -OH, -SH or -NH₂ group
R₅, R₇ and Rb are as defined above and
s₁, t₁, u₁, s₂, t₂ and u₂ same or different being integers between 0 and 10

Another object according to the invention is a method for bringing a key partner for example a ligand, a chiral moiety, another metal complex to a catalyst comprising a step of contacting said key partner with a nanohybrid according to the invention in order to turn on a specific type of activity.

Thus the method according to the invention permits to create / to complete a mono- / bi-nuclear catalyst coordination sphere.

According to the invention, a control of a 1/1 ratio between Ra and Rb or between Ra1 and Rb is made possible.

In a first mode of the invention (first mode), a ligand L1 able to coordinate with a metal M is bound to Ra (Scheme 1A) or Ra1 (Scheme 1B) ; Rb remains unmodified and may be for example an imidazole, a thiol or an amine and acts as a ligand. Rb will make it possible to complete the coordination sphere of the metal.

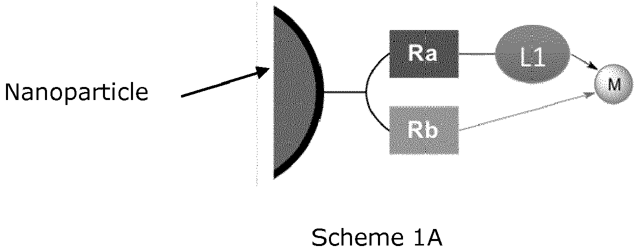

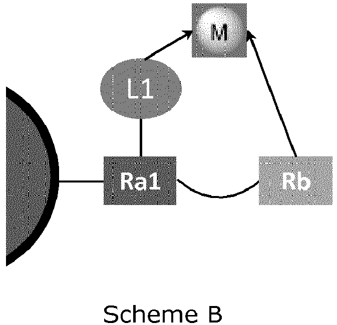

### First mode

In a second mode of the invention (second mode), a ligand L1 able to coordinate with a metal M1 is bound to Ra (Scheme 2A) or Ra1 (Scheme 2B); Rb remains unmodified and may be for example an imidazole, a thiol or an amine and acts as a ligand. Rb will coordinate a second metal (M2) to develop a binuclear catalyst or two catalytic sites (M1 and M2)

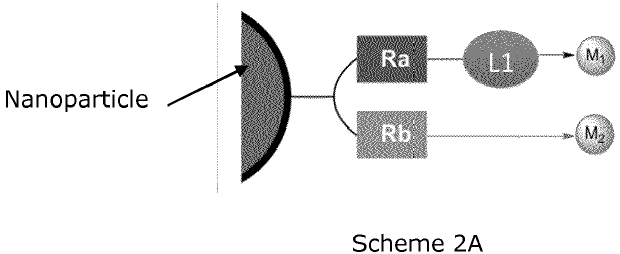

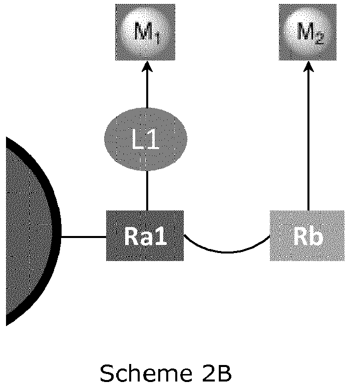

### Second mode

In a third mode of the invention (third mode), Ra (Scheme 3A) or Ra1 (Scheme 3B) and Rb are both modified in order to permit the binding of a ligand L1 and a ligand L2 respectively said ligands being able to recreate the complete coordination sphere of a unique metal (M).

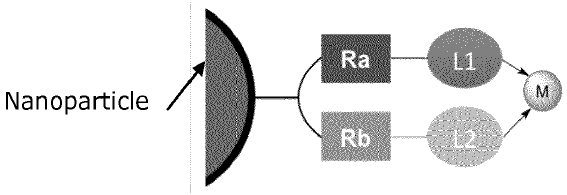

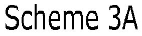

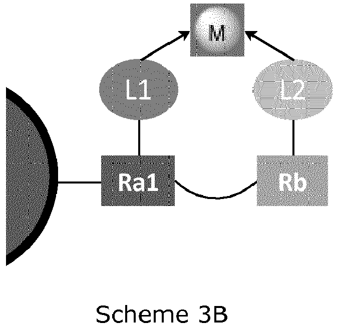

### Third mode

In a fourth mode of the invention (fourth mode), Ra (Scheme 4A) or Ra1 (Scheme 4B) and Rb are both modified in order to permit the binding of a ligand L1 and a ligand L2 respectively said ligands being able to recreate the coordination sphere of two metals (M1) and (M2) to give a binuclear catalyst or two catalytic sites.

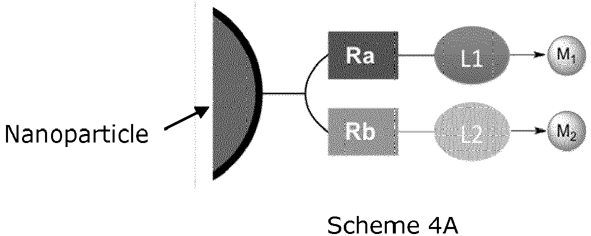

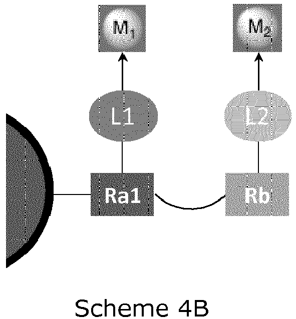

### Fourth mode

Another object according to the invention is the use of a nanohybrid according to the present invention as support for at least one catalyst.

According to the invention, the catalyst may be any catalyst known from the one in the art in particular it may be chosen in the group comprising:
- Oxidation catalysts (porphyrin or salen metallic complexes)
- Photocatalysts for alkene oxygenation (the photosensitizer and cobalt cobaloxime pair)
- Asymmetric aziridination (bisoxazoline copper complex, manganese, ruthenium salen, cobalt porphyrin with chiral pickets)
- Asymmetric hydrogenation (rhodium(I) and ruthenium(I) (*R*)-BINAP complex)
- Enantioselective Reduction catalyst (ruthenium salen complex)
- C-C bond formation coupling catalysts, such as Heck coupling catalyst (bipyridyl palladium complex), Suzuki cross-coupling catalyst (dibenzoacetate palladium complex), and Sonogashira catalyst
- Hydroformylation catalysts
- Catalysts for the cyanation of aryl aldehydes (vanadium, titanium salen complexes)
- Asymmetric transfer hydrogenation catalysts (iridium(III), rhodium(III) based cyclopentadienyl based ligands) from ketones or imines to chiral alcohols and amines
- Enzyme-based biocatalysts (lipase, oxidoreductase, glycosidase, protease...) to achieve the synthesis of enantiopure compounds (or kinetic resolution of a racemic mixture)
- Asymmetric epoxide opening (cobalt salen complex)

Another object according to the invention is a method for catalysing a chemical reaction comprising adding a nanohybrid according to the invention supporting at least one catalyst in the reaction medium.

According to the invention, nanohybrids may be used for example for oxidation, C-C coupling, hydrogenation etc. according to the catalyst supported by said nanohybrid.

Our technological solution in particular via magnetic iron-oxide-based nanohybrids also makes catalyst recovery easier with a simpler, faster and more efficient process than with catalysts immobilized on nanoparticles of silica (nonmagnetic) or on polymers. The process according to the invention does not require changing the process, simply using a magnetic recovery system such as a permanent magnet or a temporary magnet (electromagnet). This minimizes the use of solvents and the number of purification steps and addresses issues relevant to green chemistry.

This magnetic solution allows easy catalyst recovery in a solution where only the supported catalyst is present (no solvents, reagents or products), which allows either to re-engage the catalyst in a new run (easy reuse) or to treat the catalyst at the end of its life (easy recycling).

Finally, the property of being able to covalently, specifically and selectively attach one or two ligands at a single anchor point will allow the pre-organisation of the catalyst site(s) in order to counteract the limits of the supported catalysis (catalyst deactivation, lower catalytic activity and metal release).

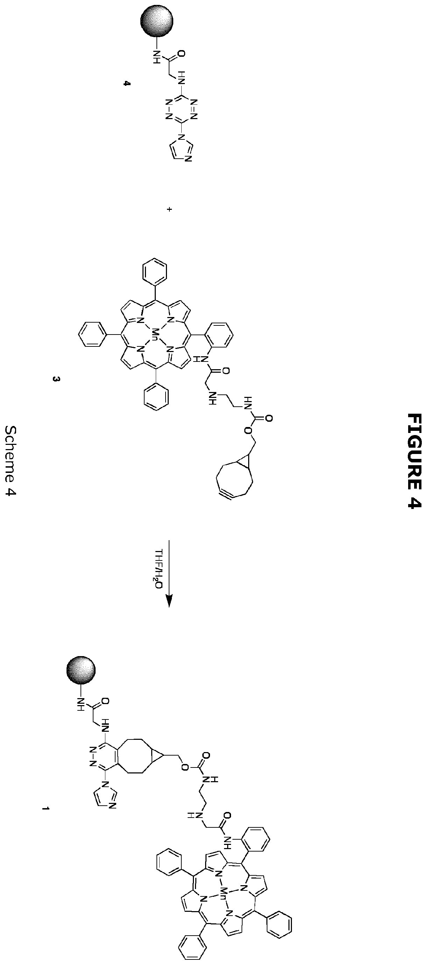

The invention will be illustrated by the examples I and II below and by figures 1 to 4.
Figure 1 illustrates the synthesis of compound 2 of formula (IIb) according to example 1.1. DIPEA: N,N-diisopropylethylamine; ACN: acetonitrile
Figure 2 illustrates the synthesis of a modified manganese porphyrin 3 according to example 1.2.
Figure 3 illustrates the synthesis of a bi-functional nanohybrid 4 according to example 1.3.
Figure 4 illustrates the immobilization of the modified manganese porphyrin 3 on the bi-functional nanohybrid 4 according to example 1.4.

### EXAMPLE I: SYNTHESIS OF AN OXIDATION CATALYST NANOHYBRID 1 FROM A MAGNETIC IRON OXIDE NANOPARTICLE, A 1,2,4,5-TETRAZINE AS BIOORTHOGONAL FUNCTION (Ra1), AN IMIDAZOL AS CHEMICAL FUNCTION (Rb) AND A BICYCLONONYNE-CONTAINING MANGANESE PORPHYRIN

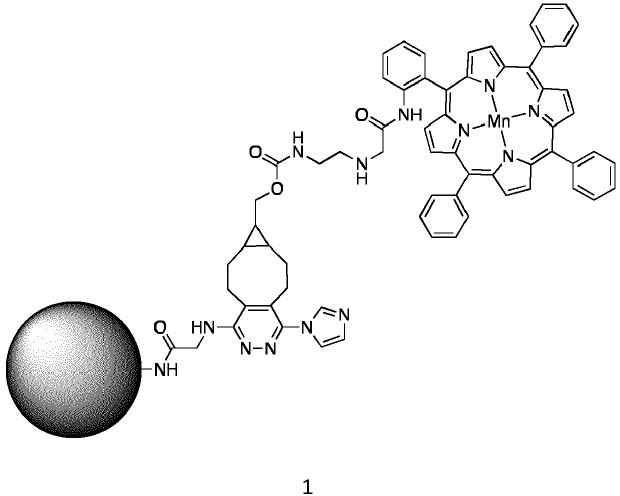

### 1.1. Synthesis of compound 2 of formula (IIb)

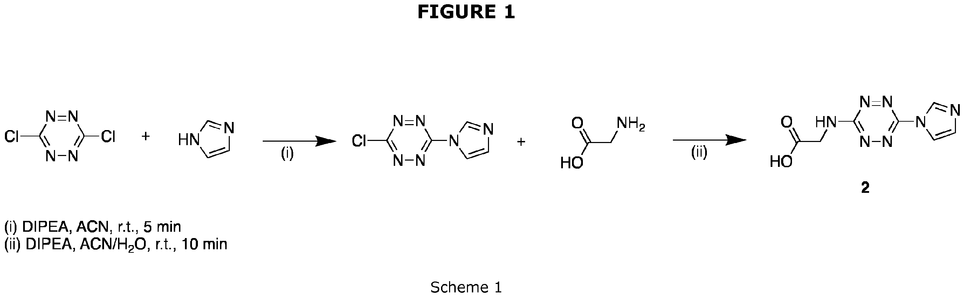

In a first step, 3,6-dichloro-1,2,4,5-tetrazine (100 mg, 0.66 mmol) is reacted with imidazole (45 mg, 0.66 mmol) and N,N-diisopropylethylamine (DIPEA) (120 µL, 0.66 mmol) in acetonitrile (ACN) (6 mL) at room temperature. After 5 minutes, the formation of the product is confirmed by TLC (ethyl acetate/dichloromethane, 2:8) and HPLC-MS. In a second step, glycine (50 mg, 0.66 mmol) solubilized in water (1mL) and DIPEA (120 µL, 0.66 mmol) are added to the reaction mixture. After 10 minutes, the formation of the final product 2 is confirmed by TLC (methanol/dichloromethane, 1:1) and HPLC-MS. Solvents are eliminated under reduced pressure and the crude final product is purified by semi-preparative HPLC to give the final product 2 according to scheme 1 given in figure 1.

### 1.2. Synthesis of a modified manganese porphyrin 3

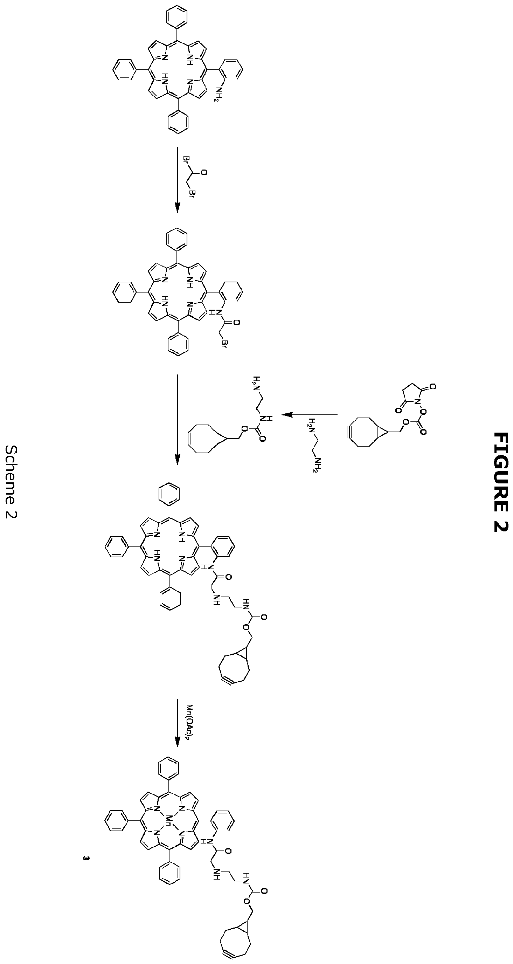

The manganese porphyrin has been synthetized according to and adapted from methods described in the literature (Org. Lett. 2004, 6, 1033-1036; J. Phys. Chem. B 2006, 110, 15955 and Synlett 1999 (1) 61-62) and modified by the introduction of a (bicyclo[6.1.0]non-4-yn-9-ylmethanol) moiety according to the following method illustrated in scheme 2 in figure 2. This function makes the specific and selective binding of the catalyst to the nanohybrid possible.

### 1.3. Synthesis of the bi-functional nanohybrid 4

The synthesis is based on the work of Jérémy PARIS (Iron oxides nanoparticles and titanate nanotubes dedicated to multimodal imaging and anticancer therapy , 2013, Université de Bourgogne)

In a first step, an -NH₂ function is introduced at the surface of the nanoparticles with (3-aminopropyl)triethoxysilane.

Bare nanoparticles were subjected to 3-aminopropyltriethoxysilane (APTES) in an equivalent mass ratio into 20 mL of a 1:1 ethanol/water mixture. The mixture was submitted to an ultrasonic treatment. The mixture was then submitted to mechanical stirring (60 rpm) during 48h. 20 mL of glycerol was then added followed by the evaporation of the ethanol/water mixture. Finally, glycerol was removed by acetone addition to the nanoparticle suspension accompanied by a magnetic decantation. Nanoparticles were finally re-suspended into ultrapure water yielding nanoparticles coating -NH₂ function.

In a second step the coupling between the nanoparticles of iron oxide and the organic compound 2 is achieved in the presence of NHS and EDC as coupling agents.

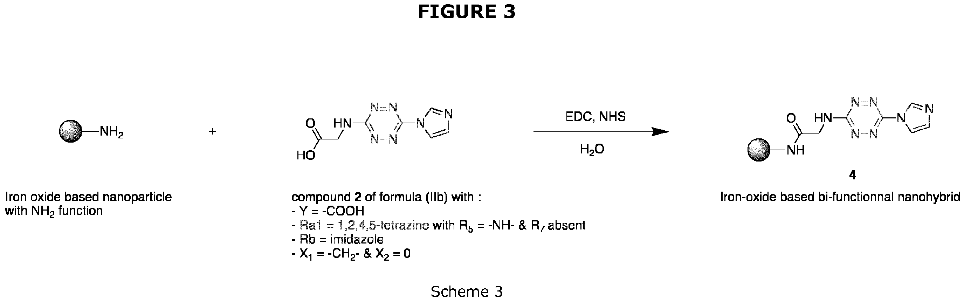

Nanoparticles coating -NH₂ groups were subjected to an equivalent mass ratio of compound 2 in the presence of *N*-(3-dimethylaminopropyl)-*N*-2-ethylcarbodiimide hydrochloride (EDC) and *N*-hydroxysuccinimide (NHS) at 2.0 and 2.2 molar equivalent respectively according to scheme 3. The mixture was magnetically stirred (500 rpm) in 30 mL of water during 48h at room temperature followed by magnetic decantation and washing with 4x50 mL water leading to the bi-functional nanohybrid 4 (see scheme 3 in figure 3).

### 1.4. Immobilization of the modified manganese porphyrin 3 on the bi-functionnal nanohybrid 4

In a third step, the covalent binding of the porphyrin is achieved via the bioorthogonal function 1,2,4,5-tetrazine of the bi-functional nanohybrid 4 and the partner bioorthogonal function (a bicyclo[6.1.0]non-4-yn-9-ylmethanol function) of the modified manganese porphyrin 3 according to scheme 4. The mixture was stirred at room temperature into 30 mL of a 1:1 tetrahydrofuran/water mixture for 15h followed by magnetic decantation and washing with 3x50 mL tetrahydrofuran and 3x50 mL water. The resulting nanohybrid 1 was characterized by both UV/Vis (band at 440 nm characteristic of a manganese porphyrin), ICP analysis (with a Mn(Porphyrin - 0.17%)/Fe(nanoparticle - 45.37% ; mₛₐₘₚₗₑ = 4.19 mg ; calculation method see Chem. Comm. 2013, 7394-7396) ratio indicating a coverage of 0.3 Mnporphyrin/nm²).

### EXAMPLE II: USE OF THE NANOHYBRID ACCORDING TO THE INVENTION AS CATALYST

The manganese porphyrin immobilised on the bi-functional nanohybrid prepared according to example I has been tested in an epoxydation reaction to examine its catalytic activities.

The reaction was examined with styrene in the presence of the immobilized manganese porphyrin and iodosylbenzene as an oxydant.

At the end of the reaction, the end product of the reaction, 2-phenyloxyran was formed as shown by both TLC and GC analyses.

In this example, the bi-functional nanohybrid consists of iron oxide nanoparticles (magnetic properties), a 1,2,4,5-tetrazine as bioorthogonal function (Ra1) and an imidazole as chemical function (Rb). The 1,2,4,5-tetrazine allows the specific and selective binding of the porphyrin ligand. The imidazole function then plays the role of second ligand by coordinating manganese, thus stabilizing manganese porphyrin and increasing its catalytic activity.

## Claims

1. Bi-functional nanohybrid comprising a nanoparticle to the surface of which are covalently coupled one or more, identical or different, groups selected from:
- either a group of formula (1) or (1') 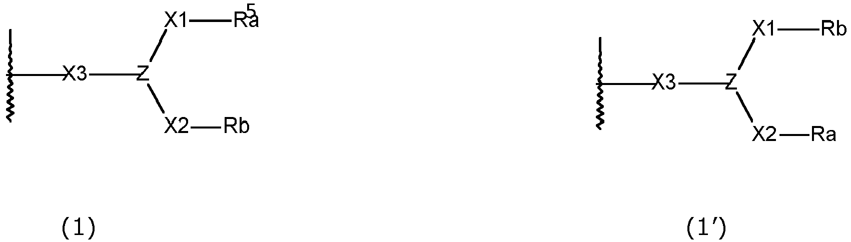 where
• Ra represents a bioorthogonal function selected from:
▪1,2,4-triazines of formula (a) 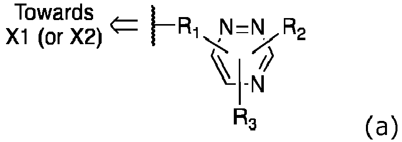
R₁, R₂ and R₃ being independently either in position 3, 5 or 6 of said 1,2,4-triazine
R₁ being either absent or an aryl or a heteroaryl groups, said groups having at least one attachment function to X1 or X2 among the positions of the cycle,
R₂ and R₃ being selected in the group comprising:
✔ hydrogen atom and halogen atoms,
✔ -CF₃, -CN, -NO₂ and
✔ aryl and heteroaryl group,
said triazine of formula (a) being bound to X1 or X2 in one of the position 3, 5 or 6 either directly (when R₁ is absent) or via R₁,
and from
▪1,2,4,5-tetrazines of formula (b) 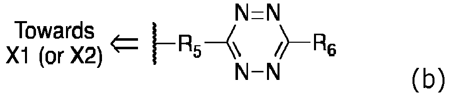
R₅ being absent or being selected in the group comprising:
✔ oxygen and sulphur atoms,
✔ -O-aryl and -S-aryl said aryl group bearing an attachment function to X1 or X2 in any free position,
✔ -NRm- with Rm being a hydrogen atom, a (C₁-C₄)alkyl group, a (C₃-C₆)cycloalkyl group, an aryl group or a heteroaryl group,
✔ -NRm-aryl said aryl group bearing an attachment function to X1 or X2 in any free position and
✔ aryl and heteroaryl group, said group bearing an attachment function to X1 or X2 in any free position.
R₆ being selected in the group comprising:
✔ hydrogen atom,
✔ (C₁-C₄)alkyl group and (C₃-C₆)cycloalkyl group,
✔ -ORm and -SRm with Rm being a hydrogen atom, a (C₁-C₄)alkyl group, a (C₃-C₆)cycloalkyl group, an aryl group or a heteroaryl group,
✔ -NRmRo with Rm and Ro being independently from each other a hydrogen atom, a (C₁-C₄)alkyl group, a (C₃-C₆)cycloalkyl group, an aryl group or a heteroaryl group and
✔ aryl and heteroaryl group,
said tetrazine of formula (b) being bound to X1 or X2 either directly (when R₅ is absent) or via R₅
• Rb represents
∘ an halogen atom,
∘ a -ORm or -SRm group with Rm being a hydrogen atom, a (C₁-C₄)alkyl group, a (C₃-C₆)cycloalkyl group, an aryl group or a heteroaryl group,
∘ a -COORm group with Rn being a hydrogen atom, a (C₁-C₄)alkyl group, a (C₃-C₆)cycloalkyl group, an aryl group or a heteroaryl group,
∘ a -NRmRo with Rm and Ro being independently from each other a hydrogen atom, a (C₁-C₄)alkyl group, a (C₃-C₆)cycloalkyl group, an aryl group or a heteroaryl group
∘ an azide group,
∘ a maleimidyl group,
∘ a (C₁-C₄)alkynyl group,
∘ an imidazolyl group,
∘ a pyridinyl group,
∘ a 1,2,4-triazine of formula (a),
∘ a 1,2,4,5-tetrazine of formula (b),
∘ a diphenylphosphinobenzoate derivative of formula (c) 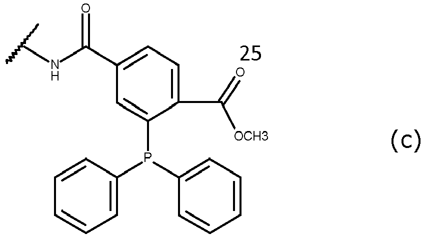 and
∘ a norbornene derivative of formula (d) 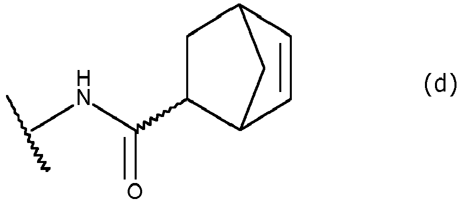 and
• X1, X2 and X3, same or different, may be absent or represent a spacer selected from (C₁-C₂₆)alkyl groups; said groups may comprise one or more oxygen atoms and/or one or more groups 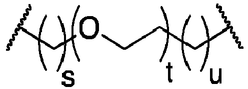 with s, t and u, same or different being integers between 0 and 10,
• Z represents an aryl group or a heteroaryl group that is at least tri-substituted by substituents allowing the attachment to X1, X2, and X3 respectively. The one skilled in the art will be able to choose said substituents in view of his general knowledge.
- either a group of formula (2) 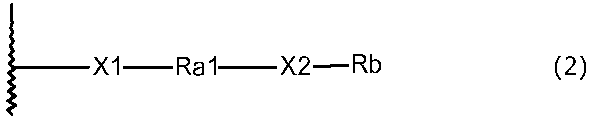 where
• Ra1 represents a bioorthogonal function selected from:
▪1,2,4-triazines of formula (e) 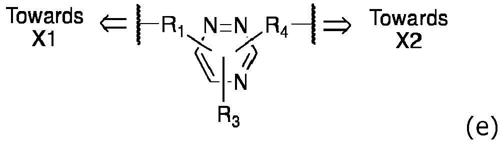
R₁, R₃ and R₄ being independently either in position 3, 5 or 6 of said 1,2,4-triazine
R₁ and R₃ being as defined above
R₄ being either absent or an aryl or a heteroaryl group, said groups having at least one attachment function to X2, among the positions of the cycle,
said triazine being bound respectively to X1 and X2 in two of the positions 3, 5 or 6 either directly (when R₁ or R₄ or both are absent) or via R₁ and R₄, respectively,
and
▪1,2,4,5-tetrazines of formula (f) 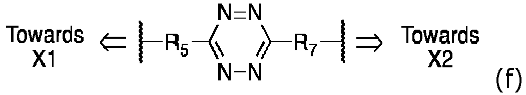
R₅ being as defined above.
R₇ being selected in the group comprising:
✔ oxygen and sulphur atoms,
✔ -O-aryl and -S-aryl said aryl group bearing an attachment function to X2 in any free position,
✔ -NRm- with Rm being a hydrogen atom, a (C₁-C₄)alkyl group, a (C₃-C₆)cycloalkyl group, an aryl group or a heteroaryl group,
✔ -NRm-aryl said aryl group bearing an attachment function to X2 in any free position and
✔ aryl and heteroaryl group said group bearing an attachment function to X2 in any free position,
said tetrazine being bound to X1 and X2 either directly (when R₅ or R₇ or both are absent) or via R₅ and R₇, respectively
and
• Rb is as defined above
• X1 and X2 are as defined above
with the proviso that Ra and Rb are mutually compatible and that the ratio between Ra and Rb or between Ra1 and Rb is equal to 1/1.

2. Bi-functional nanohybrids according to claim 1 wherein Ra in formula (1) and (1') is a 1,2,4-triazine of formula (a1) 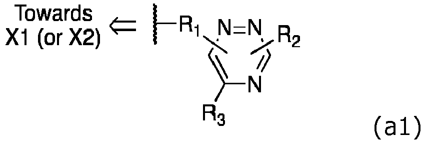 wherein
R₃ is a hydrogen atom and is in position 5 of the said 1,2,4-triazine
R₁ is selected from 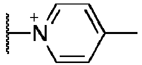 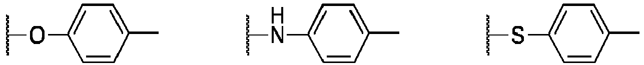 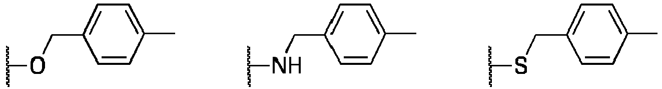 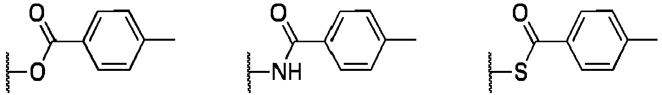
R₂ is selected from:
✔ a hydrogen atom
✔ a bromine atom, a fluorine atom
✔ a -CF₃ group, a -CN group
✔ a phenyl group optionally mono- or poly- substituted by a fluorine atom, -OCH₃ group, -CF₃ group, -NO₂ group
R₁ and R₂ being independently either in position 3 or 6 of said 1,2,4-triazine.

3. Bi-functional nanohybrids according to claim 1 wherein Ra in formula (1) and (1') is a 1,2,4,5-tetrazine of formula (b1) 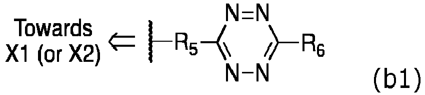 wherein
R₅ is selected from:
✔ an oxygen atom, a sulphur atom
✔ a -NH- group
✔ one of the following groups 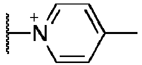 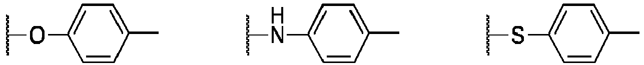 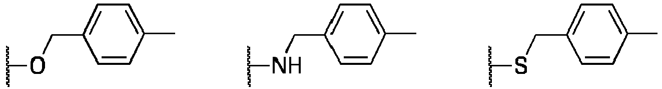 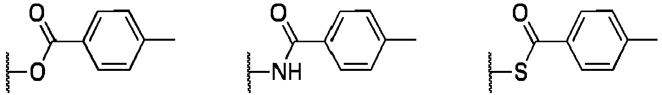
R₆ is selected from:
✔ a hydrogen atom,
✔ a methyl group,
✔ -OCH₃ and -SCH₃,
✔ -N(CH₃)₂ and
✔ a phenyl group optionally mono- or poly- substituted with a fluorine atom, -OCH₃ group, -CF₃ group, -NO₂ group.

4. Bi-functional nanohybrids according to claim 1 wherein Ra1 of formula (2) corresponds to 1,2,4-triazine of formula (e1) 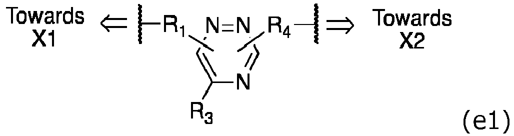 wherein
R₁ is selected from 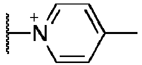 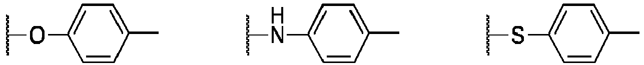 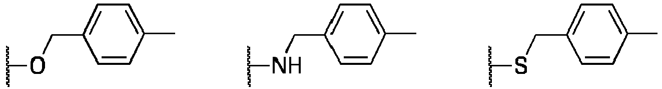 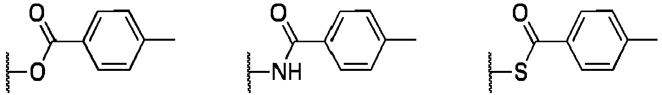
and R₃ is a hydrogen atom and is in position 5 of the said 1,2,4-triazine
R₄ is selected from 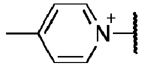 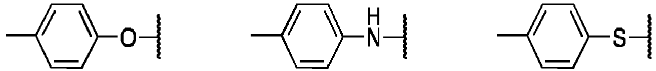 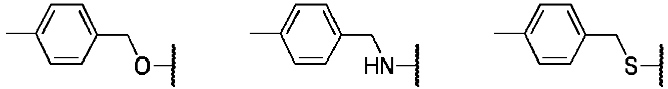 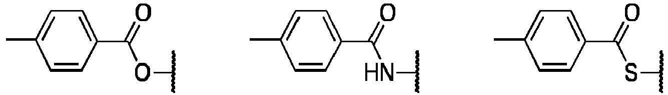
R₁ and R₄ being independently either in position 3 or 6 of said 1,2,4-triazine R₁ and R₄ being the same or different.

5. Bi-functional nanohybrids according to claim 1 wherein Ra1 of formula (2) corresponds to a 1,2,4,5-tetrazine of formula (f1) 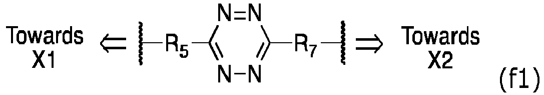 wherein
Rs is selected from:
✔ an oxygen atom, a sulphur atom
✔ a -NH- group
✔ one of the following groups 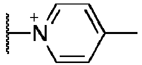 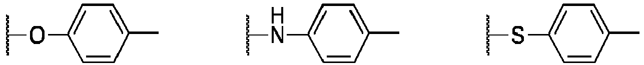 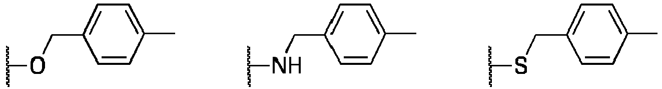 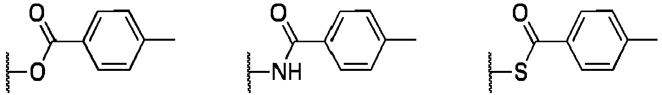
R₇ may be absent or is selected from:
✔ an oxygen atom, a sulphur atom
✔ a -NH- group
✔ one of the following groups 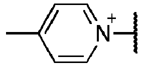 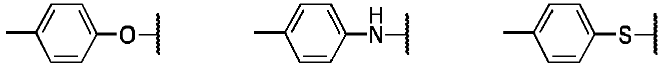 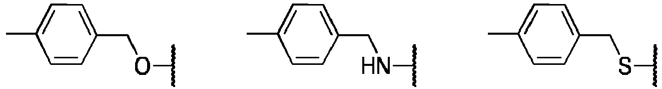 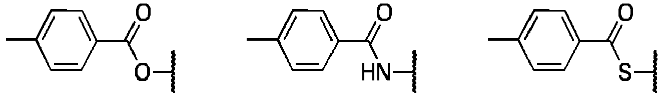
R₅ and R₇ being the same or different.

6. Method for synthesizing a bi-functional nanohybrid according to claim 1, comprising the step of attachment on a nanoparticle bearing at its surface a substituent Xa at least one compound of formula (I) 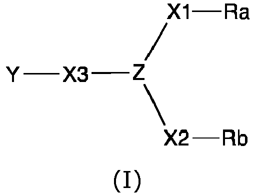 or of formula (I') 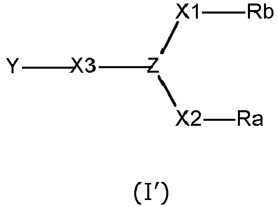 or of formula (II) 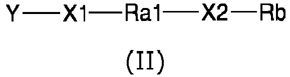 wherein
Ra, Ra1, Rb, X1, X2, X3 and Z are as defined above and
Y represents a functional group, which allows the covalent attachment to the surface of said nanoparticle via Xa and is
- either a chlorine, a bromine or an iodine atom
- either a -NRpRq group with Rp and Rq being each independently from the other a hydrogen atom, a (C₁-C₄)alkyl group or a (C₃-C₆)cycloalkyl group,
- either a carboxylic acid group,
- either an activated ester group,
- either a maleimidyl group,
- either a thiol group,
- either a hydroxyl group,
- either a tosylate group,
- either a triflate group,
- either a mesylate group,
- either an acid halide group,
- either an acid anhydride group,
- either an isocyanate group,
- either an isothiocyanate group,
- either an azide group,
- either an alkynyl group,
- or a N-heteroaryl group

7. Compound of formulas (Ia), (Ib), (Ic), (Id) or (Ie) 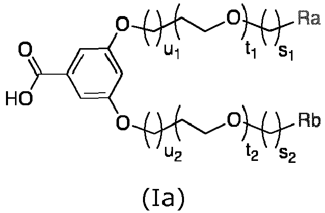 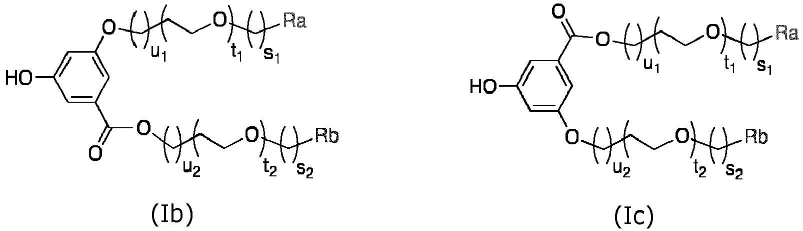 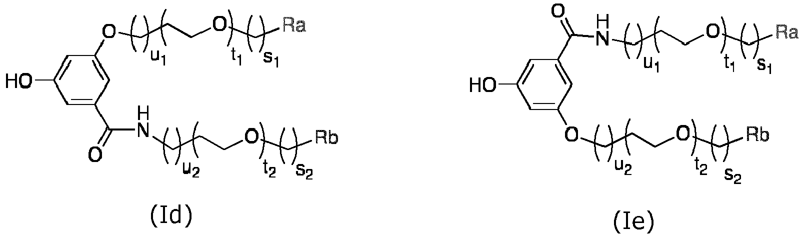 wherein
Ra represents a 1,2,4-triazine of formula (a1) or a 1,2,4,5-tetrazine of formula (b1),
Rb is as defined above,
s₁, t₁, u₁, s₂, t₂ and u₂ same or different are integers between 0 and 10,
Y is a -COOH (Ia) or a -OH (Ib), (Ic), (Id) and (Ie) and
X3 is absent.

8. Compound of formula (II) wherein Ra1 is a 1,2,4-triazine corresponds to formula (e1) and corresponding to formula (IIa) 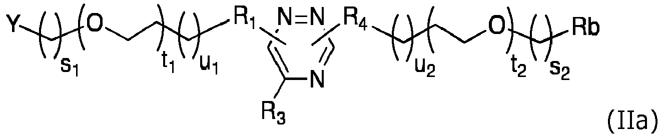 wherein
Y represents
- either a chlorine, bromine, iodine atoms
- either a -COOH group,
- either an activated ester group,
- either a maleimidyl group,
- either a -OH, -SH or -NH₂ group
R₁, R₃, R₄ and Rb are as defined above and
s₁, t₁, u₁, s₂, t₂ and u₂ same or different are integers between 0 and 10,

9. Compound of formula (II) wherein Ra1 is a 1,2,4,5-tetrazine corresponds to formula (f1) and corresponding to formula (IIb) 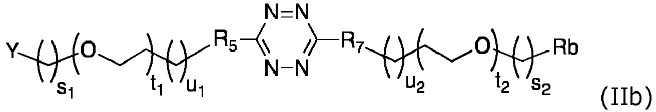 wherein
Y represents
- either a chlorine, bromine, iodine atoms
- either a -COOH group,
- either an activated ester group,
- either a maleimidyl group,
- either a -OH, -SH or -NH₂ group
R₅, R₇ and Rb, are as defined above and
s₁, t₁, u₁, s₂, t₂ and u₂ same or different being integers between 0 and 10.

10. Method for bringing a key partner selected from the group comprising a ligand, a chiral moiety or another metal complex to a catalyst comprising a step of contacting said key partner with a bi-functional nanohybrid according to claim 1 in order to turn on a specific type of activity.

11. Use of a bi-functional nanohybrid according to claim 1 as support for at least one catalyst.

12. A method for catalysing a chemical reaction comprising a bi-functional nanohybrid according to claim 1 supporting at least one catalyst in the reaction medium.
